# EUROPEAN PATENT APPLICATION

(11) **EP 4 424 680 A1**
(43) Date of publication of application: **04.09.2024**
(21) Application number: 23160024.8
(22) Date of filing: 03.03.2023
(51) Int. Cl.: C07D 317/36, C07C 68/04, C07C 69/96

(54) **CONTINUOUS-FLOW PROCESS FOR THE PRODUCTION OF GLYCEROL CARBONATE**

(71) Applicant: Université de Liège, 4000 Liège (BE)
(72) Inventor: MUZYKA, Claire, 4000 Liège (BE); MONBALIU, Jean-Christophe, 4000 Liège (BE); RENSON, Sébastien, 4000 Liège (BE)
(74) Representative: Winger

(57) **Abstract**

A continuous-flow process for the production of glycerol carbonate, wherein the method comprises reacting, in a liquid solvent in a first channel of a flow reactor, glycidol with carbon dioxide in the presence of a homogeneous nitrogen-containing organocatalyst having a p*K*ₐ of at least 15 when measured in acetonitrile, wherein the reaction is carried out at a temperature of at least 90 °C, and wherein the first channel has an inner hydraulic diameter of from 100 to 2000 µm, and a pressure in the first channel is adapted to keep the solvent liquid.

## Description

### Technical field of the invention

The present invention relates to the production of glycerol carbonate.

### Background of the invention

The production of glycerol carbonate (GLC) may have a significant impact on the chemical industry. GLC has several advantages over other, petro-based, carbonates, such as ethylene carbonates (EC) and propylene carbonates (PC), which are key electrolyte carriers in lithium batteries. The flammability of GLC is much lower than that of EC and PC, thus significantly reducing the inherent fire hazards associated with Li-based batteries. Advantageously, GLC may be formed from bio-renewables. Furthermore, GLC has applications deeply rooted in the polymer sector as blowing agent in polyurethane foams, among other cyclic carbonates. Further applications of GLC include its use as a bio-lubricant, a formulating agent, or an alternative green solvent, to name a few.

Even with its numerous applications, the global production of GLC is still limited, estimated at only around 3 Mt y⁻¹ in 2020. Nevertheless, it is expected that GLC's global production will follow a similar trend to the growth of its main building block, namely glycerol (GLY). The main reason relates to the overall inefficiency of current industrial processes. In the state of the art, there are three main synthetic routes to access GLC, either starting directly from GLY or from one of its activated derivatives, namely glycidol (GD): (A) the transesterification/alcoholysis of GLY using a carbonating agent such as dimethyl carbonate or urea, (B) the direct carbonation of GLY with carbon monoxide or carbon dioxide, and (C) the coupling of carbon dioxide with GD. Despite its effectiveness, path (A) suffers from a poor atom economy. Path (B) usually requires expensive, toxic, and depleting metal-based catalysts, as well as a large excess of additives under harsh conditions (supercritical conditions), and is often associated with low yields. Path (C) feeds upon carbon dioxide and a derivative of GLY, namely GD in the presence of various catalysts. The most usual routes to access GLC often struggle with the alleged inert nature of carbon dioxide, hence commonly leading to long reaction times under high temperature and pressure in the presence of a large excess of carbon dioxide and high catalytic loadings. Using activated substrates, such as epoxides including GD, is often considered in lieu of GLY, but still suffers from extended residence time which hampers the global efficiency of the process.

The primary literature witnessed a variety of carbonation protocols using carbon dioxide, i.e., CO₂, on a library of epoxides with homogeneous and heterogeneous catalysts under continuous flow conditions. Homogeneous catalysts often rely on binary systems based on halide salts, although halogen-free systems have also been reported. Halide salts are notoriously hard to recover and are associated with corrosion issues. Most examples of heterogeneous catalysts concerned supported ionic liquids (ILs), with the only exception of a mesoporous melamine-formaldehyde resin. Notwithstanding their effectiveness, supported ILs are cumbersome to prepare. All in all, this led to the development of a multitude of reaction conditions that are only amenable to lab scale in batch.

Only two reports specifically disclosed the carbonation of GD under continuous flow conditions, including Zanda, N., Sobolewska, A., Alza, E., Kleij, A. W., & Pericàs, M. A. Organocatalytic and halide-free synthesis of glycerol carbonate under continuous flow. ACS Sustainable Chemistry & Engineering 9 (2021) pages 4391-4397, with an heterogenous catalyst (Merrifield-immobilized 1.5.7-triazabicyclo[4.4.0]-dec-5-ene, TBD) and Valverde, D., Porcar, R., Zanatta, M., Alcalde, S., Altava, B., Sans, V., & García-Verdugo, E. Towards highly efficient continuous-flow catalytic carbon dioxide cycloadditions with additively manufactured reactors. Green Chemistry, 24 (2022) pages 3300-3308, with a 3D-printed reactor grafted with imidazolium ionic liquids. Despite their selection of a potentially ground-breaking approach, the study relies on a technological and chemical solution that only enabled lab scale applications with low overall productivities. Additionally, most, if not all, studies relied on petrobased GD.

There is thus still a need in the art for processes that address at least some of the above problems.

### Summary of the invention

It is an object of the present invention to provide a good process for the production of glycerol carbonate.

The above objective is accomplished by a process according to the present invention.

It is an advantage of embodiments of the present invention that an efficiency of the process may be good. It is an advantage of embodiments of the present invention that the E-factor of the process, which provides an estimation of the waste generated by the process, may be low. It is an advantage of embodiments of the present invention that the process may have a low energy consumption. It is an advantage of embodiments of the present invention that the process may have a high yield. It is an advantage of embodiments of the present invention that the process may have a high conversion even at a short residence time. It is an advantage of embodiments of the present invention that the process may have a high throughput. It is an advantage of embodiments of the present invention that, by using a homogeneous catalyst, the efficiency of the reaction may be good.

It is an advantage of embodiments of the present invention that the process may have a good scalability.

The present invention relates to a continuous-flow process for the production of glycerol carbonate, wherein the method comprises reacting, in a liquid solvent in a first channel of a flow reactor, glycidol with carbon dioxide in the presence of a homogeneous nitrogen-containing organocatalyst having a p*K*ₐ of at least 15 when measured in acetonitrile, wherein the reaction is carried out at a temperature of at least 90 °C, and wherein the first channel has an inner hydraulic diameter of from 100 to 2000 µm, and a pressure in the first channel is adapted to keep the solvent liquid.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

FIG. 1 is a schematic representation of a continuous-flow reactor of a process for the hydrochlorination of GLY, which may be used in embodiments of the present invention.
FIG. 2 is a schematic representation of a continuous-flow reactor of a process for the dechlorination of the hydrochlorination product of GLY, to form GD and ECH, which may be used in embodiments of the present invention.
FIG. 3 is a schematic representation of a continuous-flow reactor that is a concatenated setup of the dehydrochlorination and the hydrochlorination setups from FIG. 1 and FIG. 2, which may be used in embodiments of the present invention.
FIG. 4 is a diagrammatic illustration of the yield of GLC and GLY at different pressures and flow rates of carbon dioxide.
FIG. 5 is a diagrammatic illustration of the yield of GLC and GLY at different residence times and temperatures.
FIG. 6 is a schematic representation of a microfluidic continuous-flow reactor for forming GLC from GD and CO₂, in accordance with embodiments of the present invention.
FIG. 7 is a diagrammatic illustration of the yield of GLC at different temperatures and concentrations of the organocatalyst Barton's Base, i.e., BB.
FIG. 8 is a diagrammatic illustration of the yield of GLC at different temperatures and concentrations of the organocatalyst 1,8-diazabicyclo[5.4.0]undec-7-ene, i.e., DBU.
FIG. 9 is a schematic representation of a mesofluidic continuous-flow reactor for forming GLC from GD and CO₂, in accordance with embodiments of the present invention.
FIG. 10 is a diagrammatic illustration of the yield of GLC and GLY at different residence times and temperatures for the mesofluidic continuous-flow reactor in accordance with embodiments of the present invention.
FIG. 11 is a diagrammatic illustration of the yield of GLC as a function of time for the mesofluidic continuous-flow reactor in accordance with embodiments of the present invention.
FIG. 12 is a plot of calculated variation of Free Gibbs energy of reaction Δ*G*° and the calculated activation barrier Δ*G*^{‡} as a function of p*K*_{aH} (in acetonitrile; values from literature) of a range of organocatalysts, calculated for the first intermolecular step of the theoretically calculated reaction profile.
FIG. 13 is a plot of calculated Free Gibbs energy of reaction Δ*G*° as a function of p*K*_{aH} (in acetonitrile; values from literature) of a range of organocatalysts, calculated for the second intermolecular step of the theoretically calculated reaction profile.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. The word "comprising" according to the invention therefore also includes as one embodiment that no further components are present. Thus, the scope of the expression "a device comprising means A and B" should not be interpreted as being limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

The present invention relates to a continuous-flow process for the production of glycerol carbonate, wherein the method comprises reacting, in a liquid solvent in a first channel of a flow reactor, glycidol with carbon dioxide in the presence of a homogeneous nitrogen-containing organocatalyst having a p*K*ₐ of at least 15 when measured in acetonitrile, wherein the reaction is carried out at a temperature of at least 90 °C, and wherein the first channel has an inner hydraulic diameter of from 100 to 2000 µm, and a pressure in the first channel is adapted to keep the solvent liquid.

The organocatalyst at least contains a nitrogen atom. Preferably, the organocatalyst is a guanidine derivative, e.g., a substituted guanidine compound. In embodiments, the organocatalyst has the following chemical formula: 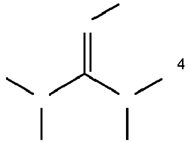 wherein
- R¹ is selected from: H, alkyl, heteroalkyl, aryl, heteroaryl, and benzyl,
- R² and R³ are either independently selected from H, alkyl, heteroalkyl, aryl, heteroaryl, and benzyl, or the radicals R² and R³, together with the nitrogen atom to which R² is bonded, the nitrogen atom to which R³ is bonded, and the carbon atom linking both said nitrogen atoms, form a five- to eight-membered ring, and
- R⁴ is independently selected from H, alkyl, heteroalkyl, aryl, heteroaryl, and benzyl, while R⁵ is independently selected from alkyl, heteroalkyl, aryl, heteroaryl, and benzyl, or the radicals R⁴ and R⁵, together with the nitrogen atom to which R⁴ is bonded, the nitrogen atom to which R⁵ is bonded, and the carbon atom linking both said nitrogen atoms, form a five- to eight-membered ring,
   with the proviso that R² and R³ on one hand and R⁴ and R⁵ on the other are not simultaneously part of a ring.

It is an advantage of these embodiments that a good selectivity towards the formation of glycerol carbonate may be achieved.

In embodiments, the alkyl may be a branched or straight C₁-C₄ alkyl. Preferred alkyls for R⁵ are methyl, ethyl, n-propyl, isopropyl, n-butyl, s-butyl, isobutyl, and t-butyl. Particularly preferred alkyls for R⁵ are methyl and t-butyl.

Each heteroalkyl comprises at least one carbon atom as well as at least one heteroatom.

In embodiments, the heteroalkyl may be a branched or straight alkyl group where one or more carbon atoms are replaced by nitrogen, oxygen, or sulfur. Preferably, it is a branched or straight C₁-C₄ alkyl group where one or more carbon atoms are replaced by nitrogen, oxygen, or sulfur.

In embodiments, the aryl is selected from phenyl, tolyl, xylyl, and naphthyl. Preferably, the aryl is phenyl.

Each heteroaryl is an aromatic ring structure, e.g., a mono-cyclic or polycyclic aromatic ring structure, that includes one or more heteroatoms.

In embodiments,
- R¹ is selected from H and methyl,
- R² and R³ are both methyl or the radicals R² and R³, together with the nitrogen atom to which R² is bonded, the nitrogen atom to which R³ is bonded, and the carbon atom linking both said nitrogen atoms, form a five-membered ring, and
- R⁴ is independently selected from H and methyl while R⁵ is independently selected from a C₁-C₄ alkyl and a phenyl, or the radicals R⁴ and R⁵, together with the nitrogen atom to which R⁴ is bonded, the nitrogen atom to which R⁵ is bonded, and the carbon atom linking both said nitrogen atoms, form a six-membered ring.

In preferred embodiments, the organocatalyst is Barton's base. The inventors have found that the process in accordance with embodiments of the present invention may have a particularly good yield and selectivity when Barton's base is used as the organocatalyst.

In embodiments, the catalyst has a p*K*ₐ of at least 20 when measured in acetonitrile. Herein, the p*K*ₐ may be as determined using a glass electrode, or may be obtained from literature. For example, Schwesinger, R., Willaredt, J., Schlemper, H., Keller, M., Schmitt, D., & Fritz, H. Novel, very strong, uncharged auxiliary bases; design and synthesis of monomeric and polymer-bound Triaminoiminophosphorane bases of broadly varied steric demand. Chemische Berichte, 127 (1994) pages 2435-2454, and Ishikawa, T. Superbases for organic synthesis: guanidines, amidines and phosphazenes and related organocatalysts. Chichester, 2009, UK: John Wiley & Sons, pages 1-336, summarize p*K*ₐ values for compounds that may be used as organocatalyst in embodiments for the present invention. In preferred embodiments, the organocatalyst has a p*K*ₐ of from 20 to 30 when measured in acetonitrile. It is an advantage of these embodiments that a high yield and selectivity may be achieved.

In embodiments, the catalyst is present in an amount of from 0.1 to 20 mol-%, with respect to the total amount, in mole, of the catalyst, the glycidol, and the carbon dioxide. In embodiments, the catalyst is present in an amount of from 0.5 to 10 mol-%, preferably from 1 to 5 mol-%, with respect to the total amount, in mole, of the catalyst, the glycidol, and the carbon dioxide. It is an advantage of embodiments of the present invention that the organocatalyst concentration may be low, while still a process with a fast reaction rate and a high conversion and selectivity may be obtained.

In embodiments, the liquid solvent may be selected from methanol, acetone, ethanol, ethylene glycol, 2-piperidineethanol, sulfolane, or amines such as amines MEA, 2-amino-2-methyl-1-propanol (AMP), diisopropylamine (DPA), methyldiethanolamine (MDEA), diethanolamine (DEA), and diisopropanolamine (DIPA), 2-butanone and n-butanol. It is an advantage of these embodiments that these solvents have good solvation properties for carbon dioxide, so that the concentration of carbon dioxide in these solvents may be high. Preferably, the solvent is 2-butanone or n-butanol. These two solvents are chosen for their low toxicity, biobased composition, and their ability to solvate glycidol - a feat that solvents of low polarity are unable to accomplish. Most preferred in 2-butanone because its propensity to react with CO2 is typically lower than solvents having hydroxyl or amine groups.

In embodiments, the flow reactor has a mixing efficiency, defined by an overall mass transfer coefficient k_{L}×a of at least 100 ×10⁻⁴ s⁻¹, preferably at least 21000 10⁻⁴ s⁻¹ where k_{L} is the mass transfer coefficient in m/s calculated by the method described in Ind. Eng. Chem. Res. 2012, 51, 16251-16262 and "a" is the effective interfacial area in m²/m³. It is an advantage of these embodiments that the solutes in the liquid solvent, i.e., the glycidol, the organocatalyst, and the carbon dioxide, may have a uniform concentration through the liquid solvent. It is an advantage of these embodiments that the reaction may be predictable and that clogging may be averted. In embodiments, the first channel is adapted for inducing said mixing. In embodiments, the flow reactor may comprise a mixer, e.g., a static mixer, that is preferably suitable for being used at the high pressures of the first channel.

In embodiments, the first channel may have a polymeric inner surface, preferably an halogenated inner surface, more preferably, a fluorinated inner surface, yet more preferably, a perfluorated inner surface. For instance, the first channel may be made of a perfluorinated material such as a perfluoroalkoxy alkane. Such channels are advantageous as they reduce the risk of clogging by polymerization of GD compared to other inner surface materials such as glass.

In embodiments, the reaction may be carried out at a temperature of from 100 °C to 170 °C, preferably from 110 °C to 160 °C, more preferably from 115 °C to 149 °C, and even more preferably from 120 °C to 145°C. The inventors have observed that using a flow reactor to provide good mixing allows for the reaction to be carried out at high temperatures (90°C or above) without leading to polymerization of polyglycerol, which is usually expected at these temperatures. Indeed, by using the continuous-flow process of embodiments of the present invention, clogging of the first channel, by said polymerization in a glass first channel, may be prevented for temperatures of up to 150 °C. Furthermore, although these high temperatures are associated with a low solubility of carbon dioxide, the inventors have unexpectedly found that a stoichiometric amount of carbon dioxide, dissolved in the liquid solvent, may suffice to have a fast and efficient reaction. Therefore, the continuous-flow process may proceed at these high temperatures, resulting in a fast reaction with a high yield.

The flow reactor may comprise means, e.g., a thermoregulator, for setting the temperature in the first channel. Said means may, for example, comprise a heating element and a temperature sensor.

The flow reactor may comprise means, e.g., a thermoregulator as above or another thermoregulator, for vaporizing the CO₂ by heating the corresponding gaseous feed.

In embodiments, the pressure in the first channel is at least 5 bar, preferably at least 10 bar, such as from 10 to 20 bar. Solvents providing good solubility for carbon dioxide, may have a boiling point below the temperature used in the reaction in accordance with embodiments of the present invention. For example, the boiling point, at a standard pressure of 1 atm, of 2-butanone is 79 °C and that of n-butanol is 117.7 °C. By increasing the pressure in the first channel, the boiling point of the solvent may be increased to a temperature above the temperature at which the reaction is performed.

The flow reactor may comprise means for pressurizing the first channel. For example, the flow reactor may comprise a pressure regulator, such as a backpressure regulator, typically located downstream of the first channel. The flow reactor may comprise a pressure sensor for detecting the pressure in the first channel. The flow reactor may comprise a pressure sensor upstream of the first channel to monitor a possible early clogging of a feed channel.

The first channel has an inner hydraulic diameter of from 100 to 2000 µm, that may define a fluidic pathway. The hydraulic diameter can typically be calculated by D_{H} = 4A/P, wherein A is the cross-sectional area of the channel and P is the wetted perimeter of the cross-section, i.e., the perimeter of the cross-sectional area that is "wet", i.e., in contact with the liquid solvent. It is an advantage of these embodiments that mixing of the compounds dissolved in the liquid may be good. Good mixing may prevent polymerization reactions, which could lower the yield of the reaction in the first channel and could result in clogging of the first channel. It is an advantage of these embodiments that the temperature gradient across the cross-section of the first channel may be low. Thereby, good and uniform reaction conditions may be obtained throughout the complete first channel.

In embodiments, the carbon dioxide is introduced in the first channel at a flow rate such that from 0.5 to 1.5, preferably from 0.9 to 1.5, more preferably from 1.0 to 1.5 equivalents of carbon dioxide is present with respect to glycidol. It was surprisingly found by the inventors that a higher flow rate, such that more than 1.5 equivalents of carbon dioxide is present with respect to glycidol, reduced the conversion percentage of glycidol to glycerol carbonate. In embodiments, the flow reactor comprises a gas flow controller for allowing the carbon dioxide to enter directly in the fluidic pathway at a controlled flow rate.

In embodiments, the residence time of the liquid solvent, in which the glycidol, carbon dioxide, and organocatalyst are dispersed, e.g., dissolved, in the first channel is from 1 second to 1 hour, preferably from 30 seconds to 20 minutes, more preferably from 1 minute to 10 minutes. Herein, the residence time means a mean time that the liquid solvent is present in the first channel and may be calculated by, for example, dividing the internal volume of the first channel by the total flow rate of gas and liquid introduced into the first channel. It is an advantage of embodiments of the present invention that the reaction may be fast, which may be used to achieve a high throughput. For example, the inventors have achieved a high conversion and selectivity at a residence time of less than 30 seconds in a process in accordance with embodiments of the present invention.

In embodiments, the process may further comprise a step, prior to the reaction of glycidol with carbon dioxide, of producing the glycidol by:
reacting glycerol with hydrochloric acid in a second channel of the flow reactor, wherein the second channel has an inner hydraulic diameter of from 100 to 2000 µm, thereby forming a reaction mixture, then reacting the reaction mixture with a base having a pKa lower than 15, and preferably lower than 11. An inorganic base, such as NaOH, KOH, Na₂CO₃, or NaHCOs may be used. The reaction mixture is reacted in a third channel of the flow reactor, wherein the third channel has an inner hydraulic diameter of from 100 to 2000 µm, thereby producing the glycidol.

In embodiments, an amount of from 1 to 20, preferably from 3 to 10, equivalents of hydrochloric acid, typically in water, is introduced in the second channel with respect to glycerol. In embodiments, a catalyst, such as acetic acid, may be added. In embodiments, an amount of from 0.1 to 1, preferably from 0.2 to 0.5, equivalents of acetic acid is introduced in the second channel with respect to glycerol. In embodiments, the temperature in the second channel is from 120 °C to 180 °C, preferably from 140 °C to 160 °C. The pressure in the second channel is adapted to keep the water in the second channel liquid. In embodiments, the pressure in the second channel is from 5 bar to 30 bar, preferably from 10 to 25 bar.

In embodiments, an amount of from 1 to 5, preferably from 1 to 3, equivalents of sodium hydroxide, typically in water, is introduced in the third channel, with respect to the hydrochloric acid in the reaction mixture. In embodiments, the temperature in the third channel is from 10 °C to 50 °C, preferably from 20 °C to 30 °C.

In embodiments, an outlet of the second channel is fluidically connected to an inlet of the third channel. In embodiments, an outlet of the third channel is fluidically connected to an inlet of the first channel. In some embodiments, the liquid solvent used in the first channel is water. In these embodiments, the outlet of the third channel may be directly connected to the inlet of the first channel. Otherwise, extraction of the glycidol from the reaction products at the outlet of the third channel may be performed, which may be subsequently introduced into the first channel, e.g., dissolved in the liquid solvent.

Glycerol may be obtained from a plant source, such as soybeans or palm. It is an advantage of these embodiments that the process of producing glycerol carbonate may make use of compounds extracted from a biological source, and hence have a low environmental impact.

### Optimizing a continuous-flow process for the production of glycerol carbonate

Capitalizing on the unmet needs within the current global context, the inventors of the present invention sought to develop a concrete solution for the production of GLC from biobased GLY through the intermediate formation of GD under scalable and intensified process conditions. Computational chemistry and applied organic synthesis are combined to set solid foundations whereupon such process would thrive.

In what follows, a two-step process for forming glycerol carbonate (GLC) from biobased glycerol (GLY) is presented. Herein, the term "biobased" is understood to mean that the compound originates from a precursor that may be extracted from a biological source, e.g., from a plant source.

A first step aimed at intensifying, i.e., improving the efficiency and reaction rate of, the preparation of biobased glycidol (GD) starting from GLY. A second step used said biobased GD for its coupling, i.e., reaction, with CO₂ to form GLC in the presence of a homogeneous catalyst. A range of organocatalysts displaying a large range of p*K*_{aH} values were screened for determining the most suitable catalyst candidate. Structure reactivity relationships were established to maximize the reaction selectivity, conversion and output. The experimental work is supported with a Density Functional Theory (DFT) study for rationalizing the mechanism. A correlation was devised, i.e., derived, between the p*K*_{aH} of the catalysts and the conversion to GLC. Most notably, the identification of the best catalyst candidate led to unprecedented reaction conditions relying on a stoichiometric amount of CO₂, a low catalyst loading (1 mol%) and extremely fast reaction (< 1 min) with very high conversion and selectivity. The scalability of the process was also validated, providing good results for the preparation of GLC with a high output at pilot scale (3.6 kg day⁻¹).

### Continuous flow production of GD from GLY

First, an integrated production scheme using biobased GLY for forming GD was designed. Previously, an optimized and intensified process for the continuous production of epichlorohydrin (ECH) starting from GLY based on a chlorination/dechlorination sequence was reported in Morodo, R., Gerardy, R., Petit, G., & Monbaliu, J. C. M. Continuous flow upgrading of glycerol toward oxiranes and active pharmaceutical ingredients thereof. Green Chemistry 21 (2019) pages 4422-4433. The process is very appealing since it only uses GLY, concentrated aqueous HCl and aqueous NaOH. The downstream section of the process comprises a selective in-line extraction, eventually leading to the selective recovery of ECH and GD.

This process was revisited and reoptimized to now favour the formation of GD for feeding the needs of the carbonation step.

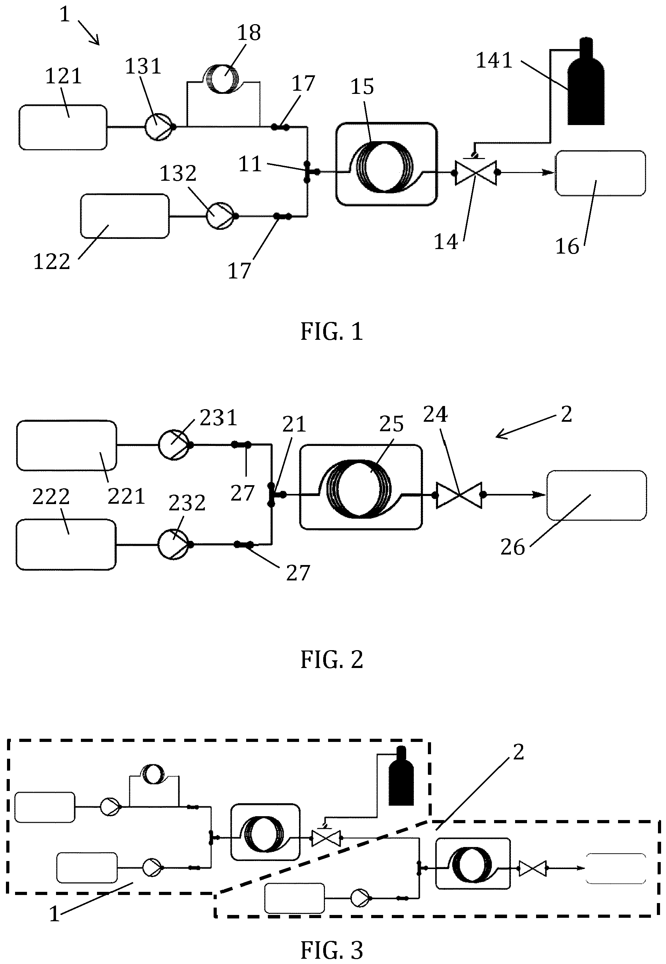

The microfluidic flow reactor 1 that was used optimizing the hydrochlorination of glycerol is shown in FIG. 1. The microfluidic flow reactor 1 contained modular coil assemblies constructed with high purity PFA capillaries (1.58 mm of outer diameter and 800 µm of internal diameter) equipped with Super Flangeless nuts and ferrules (IDEX/Upchurch Scientific). The flow reactor 1 contained a mixer 11, namely a PEEK high-pressure static mixing assembly, for mixing liquids from liquid sources 121 and 122. The liquids from liquid sources 121, 122 were introduced towards the mixer with high force Chemyx Fusion 6000 syringe pumps 131, 132 equipped with Stainless Steel syringes and check valves on each inlet (IDEX/Upchurch Scientific). Between each liquid source 121, 122 and the mixer 11, the flow reactor 1 contained an in-line check valve 17 for preventing the liquids or the mixture formed in the mixer 11 to flow back to the liquid sources 121, 122. Furthermore, the tubing between the first liquid source 121 and the mixer 11 was connected to an injection loop 18. The flow reactor 1 further contained a second channel 15, for reacting the compounds of the mixture formed by mixing the liquids in the mixer 11, and that is fluidically coupled to the mixer 11. Thermoregulation of the second channel 15 was performed with a Heidolph MR Hei-TEC^{®} equipped with a Pt-1000 temperature sensor. A dome-type backpressure regulator 14 (Zaiput Flow Technologies - BPR-1000) was utilized to set the pressure in the second channel 15 and was connected to a compressed nitrogen tank 141. Reaction products were collected at an outlet 16.

Conversions and yields for the hydrochlorination process, as well as for the dechlorination process described below, were determined by Gas Chromatography (GC) coupled to Flame ionization Detection (GC-FID). Selectivity was calculated as the ratio between the yield and the conversion. GC quantification was performed using n-butanol as internal standard, as well as commercial references of 3-chloropropane-1,2-diol, propane-1,2,3-triol, 2-chloropropane-1,3-diol, 1,3-dichloropropan-2-ol and 2,3-dichloropropan-1-ol.

The hydrochlorination step of GLY leads to a mixture, that was collected at the outlet 16, of monochlorohydrins (MCH) and dichlorohydrins (DCH). The hydrochlorination of GLY was optimized in the presence of acetic acid (AcOH) as a catalyst for maximizing the selectivity toward the formation of the corresponding MCH, including 3-chloropropane-1,2-diol (3-MCH) and 2-chloropropane-1,3-diol (2-MCH). Upon optimization, it appeared that 0.3 equiv. AcOH and 6 equiv. HCl (as a concentrated aqueous solution), introduced from the first liquid source 121, achieved the highest conversion of GLY (at 89 %), introduced from the second liquid source 122, as well as the best selectivity toward MCH (93%), at 140 °C and 10 bar for 20 min residence time.

Despite these promising results, additional optimization was performed. The temperature (up to 160 °C) in the second channel 15 was increased to shorten the residence time of the mixture in the second channel 15. A counterpressure of 20 bar was adopted downstream the hydrochlorination reactor to maintain the reaction mixture in the liquid phase. Residence times from 30 to 120 s were next assessed, and the corresponding results are presented in Table 1. It became clear that increasing the residence time to 120 s had a positive impact on the conversion of GLY (76 to 95%), as well as on the selectivity on the formation of undesired dichlorhydrins (DCH) from 15 to 29% (see Entries 1 and 8 in Table 1). Entry 4 is a good compromise for a high conversion of GLY toward MCH (79% combined selectivity) at 80 s of reaction time and at 160 °C.

Table 1: Optimization of the hydrochlorination of GLY under continuous flow conditions comprising substrate conversion and selectivity toward MCH (%) and DCH (%). Herein, 6 equiv. HCl and 0.3 equiv. AcOH were introduced for 1 equiv. GLY. The temperature of the second reactor was 160 °C. The pressure of the second reactor was 20 bar. The GLY conversion and the MCH and DCH yields were determined by GC-FID with butan-1-ol as int. std. and selectivity calculated as the ratio between the yield and the conversion.

| Entry | t (s) | GLY conv (%) | MCH yield (%) | DCH yield (%) |
|---|---|---|---|---|
| 1 | 30 | 76 | 58 | 15 |
| 2 | 60 | 86 | 68 | 15 |
| 3 | 70 | 91 | 71 | 18 |
| 4 | 80 | 89 | 79 | 19 |
| 5 | 90 | 90 | 74 | 20 |
| 6 | 100 | 92 | 74 | 20 |
| 7 | 110 | 93 | 76 | 24 |
| 8 | 120 | 95 | 72 | 29 |

As such, the selectivity of the process was tuned towards MCH by using 0.3 equiv. of acetic acid (AcOH) and 6 equiv. of concentrated aqueous HCl. These conditions achieved the highest conversion of GLY (89 %) as well as the best selectivity toward MCH (79%) within 80 s of residence time at 160 °C (20 bar of counter-pressure). This clearly demonstrated the successful intensification in comparison with the former process before the additional optimization (that used a temperature 140 °C, a reaction time of 20 min and a pressure of 8 bar).

The dechlorination step was also revisited for both intensifying the process and optimizing the selectivity toward GD. The setup for the dehydrochlorination of reactional crude obtained from the hydrochlorination of glycerol is shown in FIG. 2. The microfluidic flow reactor 2 contained modular coil assemblies constructed with high purity PFA capillaries (1.58 mm of outer diameter and 800 µm of internal diameter) equipped with Super Flangeless nuts and ferrules (IDEX/Upchurch Scientific). The flow reactor 2 contained a mixer 21, namely a PEEK high-pressure static mixing assembly, for mixing liquids from liquid sources 221 and 222. The liquids from liquid sources 221, 222 were introduced towards the mixer with high force Chemyx Fusion 6000 syringe pumps 231, 232 equipped with Stainless Steel syringes and check valves on each inlet (IDEX/Upchurch Scientific). Between each liquid source 221, 222 and the mixer 21, the flow reactor 2 contained an in-line check valve 27 for preventing the liquids or the mixture formed in the mixer 21 to flow back to the liquid sources 221, 222. The flow reactor 2 further contained a third channel 25, for reacting the compounds of the mixture formed by mixing the liquids in the mixer 21, and that is fluidically coupled to the mixer 21. Thermoregulation of the third channel 25 was performed with a HeidolphMR Hei-TEC^{®} equipped with a Pt-1000 temperature sensor. The downstream pressure was regulated with a pressure regulator 24 that was a dome-type backpressure regulator (BPR-10, Zaiput Flow Technologies) set at the working pressure with a cylinder containing compressed nitrogen. Reaction products were collected at an outlet 26.

The MCH and DCH formed in the flow reactor of FIG. 1 was introduced as a first liquid source 221. A liquid containing 4 M solution of NaOH, corresponding to 1.5 equiv. of NaOH (per HCl equivalent), was introduced as a second liquid source 222. The dechlorination step was assessed with 3 different residence times (30, 60 and 120 s) in the presence of the 1.5 equiv. of NaOH (per HCl equivalent - at room temperature) to trigger the dechlorination step at room temperature. Results indicated that the complete conversion of all MCH and DCH species into their corresponding epoxides, namely, GD and ECH, was reached for each of the three residence times.

Reference is made to FIG. 3, which shows the concatenation of the flow reactor 1 of FIG. 1 for the hydrochlorination reaction and the flow reactor 2 of FIG. 2 for the dechlorination reaction. Herein, the reaction products of the hydrochlorination reaction are directly (e.g., without further purification) used for the dechlorination reaction. Concatenation of both steps, i.e., reactions, successfully led to GD in 75% yield, with ECH as a side product (17%). This is also depicted below.

### a. Step 1 : hydrochlorination of GLY to MCH and DCH

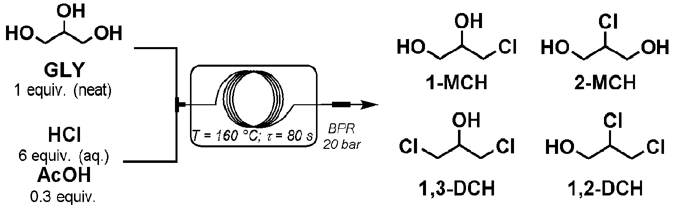

### b. Step 2 : dechlorination of MCH and DCH toward GD and ECH

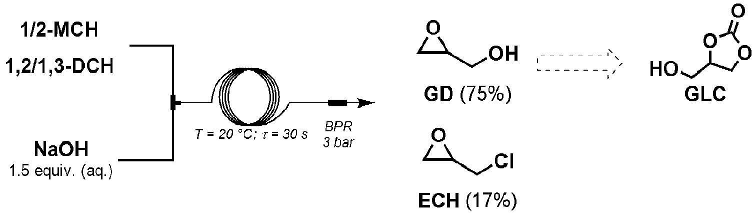

### Preliminary optimization toward the intensification of GD carbonation under flow conditions

Experiments for optimizing the continuous flow reaction of GD with CO₂ in presence of an organocatalyst were performed. A thorough investigation of elementary parameters influencing gas-liquid reactions under continuous flow conditions was undertaken. As a model catalyst, 1,5,7-triazabicyclo[4.4.0]dec-5-ene (TBD) was selected. In our approach, since the final goal was to demonstrate potential for industrial applications, CO₂ was fed into the flow reactor (either a micro reactor comprising perfluoroalkoxy alkane (PFA) capillaries or a commercial mesofluidic Corning^{®} AFR^{™} G1 Reactor) using a gas flow controller, and, furthermore, homogenous catalysis was used. The preliminary optimization started in 2-butanone (MEK) as a solvent known for its excellent CO₂ solvation properties.

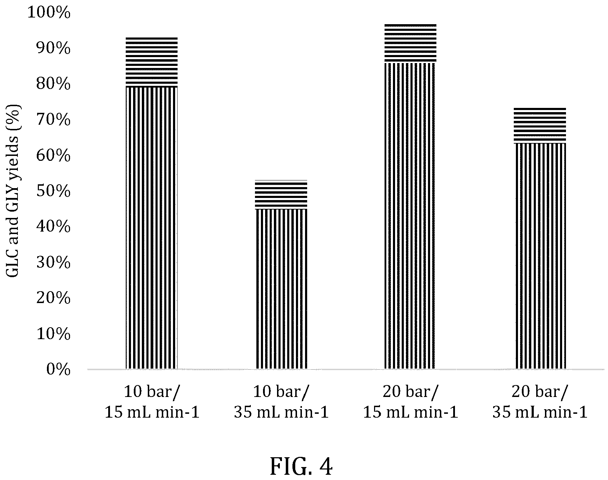

The influence of temperature, counter-pressure and CO₂ flow rate on reaction outcome were assessed with 5 mol% of TBD as a model organocatalyst. The results are summarized in Table 2. Increasing the temperature has a positive impact to boost substrate conversion, ranging from 10-25 % at 60 °C to 53-97 % at 100 °C (see for example Entry 8 in Table 2). For determining whether downstream counter-pressure has a significant impact on the outcome of the carbonation reaction, various data points were generated with the same flow rate and temperature gave similar conversion in GD, and selectivities toward GLC regardless of the downstream counter-pressure (10 or 20 bar). Reference is made to FIG. 4, which summarizes the evolution of GLC and GLY yields for a temperature of 100 °C, testing two pressures (10 and 20 bar) and two CO₂ flow rate (15 and 35 mL_{N} min⁻¹). FIG. 4 furthermore shows that a higher conversion can be obtained with a flow rate of 15 mLrr min⁻¹ of CO₂, as measured on mass balance of GLC, GLY and non-converted GD. Experiments at 10 and 20 bar show comparable yields, namely 79% at 10 bar and 86% at 20 bar for the formation of GLC, and 14% at 10 bar and 11% at 20 bar for the formation of GLY. This shows that a maximum solubility of CO₂ is already reached in the reaction medium. It can therefore be concluded that a downstream counterpressure of 10 bar already enables maximal solubilization of CO₂ in MEK. Referring back to Table 2, contrastingly to the counter-pressure, the excess of CO₂ vs GD had a pronounced impact on both the conversion and selectivity. Decreasing the gaseous flow rate from 35 (corresponding to 3.5 equiv. with respect to GD), 15 (1.5 equiv.) and 10 mLrr min⁻¹ (1 equiv.), respectively, increased the conversion of GD toward GLC.

**Table 2: Preliminary optimization of the continuous flow synthesis of GLC.**

| Entry | T (°C) | P (bar) | Flow rate CO₂ (mL_{N} min⁻¹) | GLY conv. (%) | GLC yield (%) |
|---|---|---|---|---|---|
| 1 | 60 | 10 | 15 | 16 | 16 |
| 2 | 60 | 10 | 35 | 10 | 10 |
| 3 | 60 | 20 | 15 | 25 | 25 |
| 4 | 60 | 20 | 35 | 12 | 12 |
| 5 | 100 | 10 | 10 | 94 | 83 |
| 6 | 100 | 10 | 15 | 93 | 79 |
| 7 | 100 | 10 | 35 | 53 | 45 |
| 8 | 100 | 20 | 15 | 97 | 86 |
| 9 | 100 | 20 | 35 | 73 | 63 |

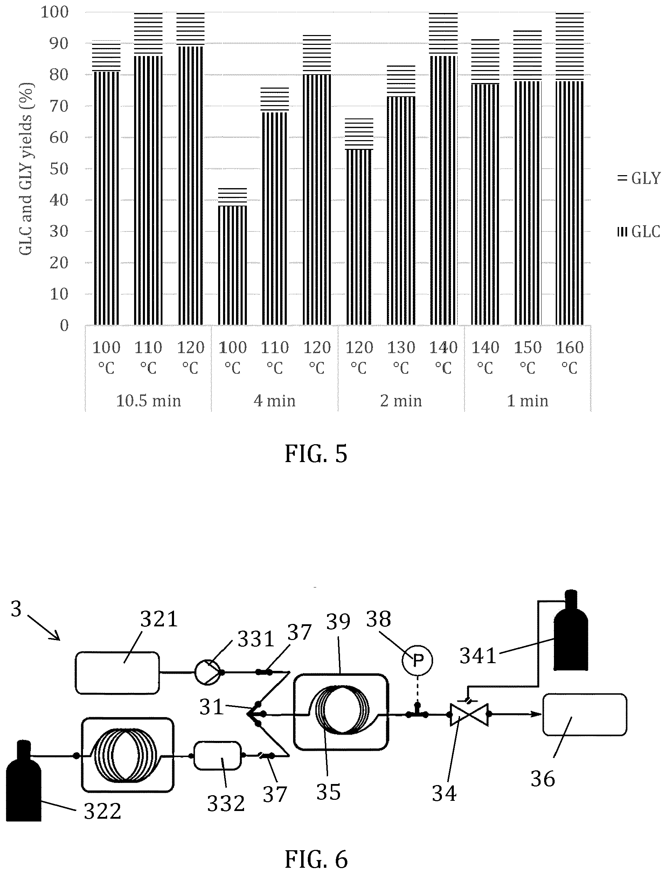

Next, the impact of the residence time on the distribution of reaction products studied. Reference is made to FIG. 5, wherein the parts of the bars having vertical stripes represent the yield of GLC and the parts of the bars having horizontal stripes represent the yield of GLY. These experiments clearly demonstrate that a high conversion of GD and a high yield in GLC are both achievable within unexpectedly short residence times when higher temperatures are used. For every residence time assessed, increasing the temperature enhanced the yield of GLC, yet it was also counterbalanced with an increase of GLY as a side product. At 10.5, 4 and 2 minutes of residence time, the yield in GLC increased from 81 at 100°C to 89% at 120°C, from 38% at 100°C to 80% at 120°C, and from 56% at 120°C to 86% at 140°C, respectively. The only exception lies with the experiments carried out within 1 min of residence time, where the yield of GLC plateaued at 77-78% at reaction temperature of from 140°C to 160°C, whereas a significant increase in yield of GLY was observed, namely from 15% at 140°C to 22% at 160°C. Therefore, the trial at 140 °C and 2 min residence time (at a pressure of 10 bar and a flow rate of 10 mLrr min⁻¹) was identified as optimum conditions, compromising both yield and selectivity under intensified conditions, e.g., at a short residence time.

### Screening of organocatalysts

Using the optimum conditions above-reported, a library of potential nitrogen-containing homogeneous catalysts was investigated. The series of homogeneous catalysts featured common organic bases including pyridine (py), triethylamine (TEA), 1,8-bis(dimethylamino)naphthalene (Proton Sponge, PS), 1,1,3,3-tetramethylguanidine (TMG), 1,8-diazabicyclo[5.4.0]undec-7-ene (DBU), 2-*tert*-butyl-1,1,3,3-tetramethylguanidine (Barton's base, BB), *tert*-octylimino-tris(dimethylamino)phosphorane (P₁-*t*-Oct), as well as 2,8,9-trimethyl-2,5,8,9-tetraza-1-phosphabicyclo- [3.3.3]undecane (Verkade's base, VB).

Reference is made to FIG. 6. To obtain a good comparison, all selected bases were evaluated under the same process conditions and using the same microfluidic reactor 3. For the homogeneous carbonation of glycidol at microscale, the microfluidic reactor 3 contained modular coil assemblies constructed with high purity PFA capillaries (1.58 mm of outer diameter and 800 µm of internal diameter) equipped with Super Flangeless nuts and ferrules (IDEX/Upchurch Scientific). The microfluidic reactor 3 contained a mixer 31, namely a PEEK high-pressure static mixing assembly. A liquid feed, containing a liquid solvent, GD, and the homogeneous nitrogen-containing organocatalyst, was introduced from a liquid source 321, and pumped towards the mixer 31 using a pump 331 that was a Syrris Asia Syringe Pump. CO₂ was introduced from a CO₂ cylinder 322, and the flow rate of CO₂ towards the mixer 31 was controller with a flow controller 332, in particular, a Bronkhorst EL FLOW Prestige mass flow controller. At the mixer 31, the CO₂ and the liquid feed were mixed. Check valves 37 (IDEX/Upchurch Scientific) were located between the pump 331 and the mixer 31, and the flow controller 332 and the mixer 31. The downstream pressure was regulated with a dome-type backpressure regulator 34 (BPR-10 Zaiput Flow Technologies), wherein the pressure may be increased by introducing nitrogen from a nitrogen cylinder 341. A homemade pressure sensor 38 (0-35 bar) was added upstream of the backpressure regulator 34 to monitor the stability of the pressure in the first channel 35 in which the carbonation reaction, i.e., the reaction between CO₂ and GD, was induced. Thermoregulation of the reactor, i.e., the first channel 35 was performed with thermoregulator 39, comprising a Heidolph MR Hei-TEC^{®} equipped with a Pt-1000 temperature sensor. Reaction products from the reaction performed in the first channel 35 were obtained at an outlet 36.

Conversions and yields for the carbonation reaction were determined by ¹H NMR spectroscopy conducted on a high field Bruker Avance spectrometer 400 MHz in d³-MeCN. 1,3,5-trimethylbenzene was used as internal standard. Commercial references of 2,3-epoay-1-propanol (GD), 4-(hydroxymethyl)-1,3-dioxolan-2-one (GLC), propane-1,2,3-triol (GLY) and 2-butanone (MEK) were used to perform peaks assignment on a ¹H NMR spectrum obtained from a typical reactional crude. Given that all signals arising from the ¹H spectrum were identified and assigned, a mass balance could be calculated for the determination of both the conversion as well and yields of all products. Quantification was done using NMR as it is a non-destructive method of analysis that ensures the observation of all species according to their distribution at the end of the experiment, unlike GC methods which can potentially alter the reactional medium during analysis. For each set of process parameters, three aliquots of the reactor effluent were subsequently collected over a period of 1 min. Yields and conversions reflect the average values of the triplicate and standard deviation (SD) is provided.

The amount of organocatalyst that was used was 5 mol% with respect to the total amount of the organocatalyst, GD and the carbon dioxide. Evaluation of the selected bases under standardized process conditions allowed to determine the impact of both basicity and steric hindrance. Table 3 summarizes the organocatalysts that were investigated here, wherein the p*K*_{aH} values were taken from Schwesinger, R., Willaredt, J., Schlemper, H., Keller, M., Schmitt, D., & Fritz, H. Novel, very strong, uncharged auxiliary bases; design and synthesis of monomeric and polymer-bound Triaminoiminophosphorane bases of broadly varied steric demand. Chemische Berichte, 127 (1994) pages 2435-2454, and Ishikawa, T. Superbases for organic synthesis: guanidines, amidines and phosphazenes and related organocatalysts. Chichester, 2009, UK: John Wiley & Sons, pages 1-336).

**Table 3: Overview of investigated homogeneous nitrogen-containing organic bases for the coupling of biobased GD with CO₂ using standardized conditions (2 min of residence time, 140 °C, 10 bar, 5 mol%). The values in the column under pK_{BH}+(MeCN) are indicative values for the experimental pK_{aH} of the catalyst in MeCN that were previously reported in literature. The yield of GLC was determined by ¹H NMR. The formation of GLY may occur through the hydrolysis of GLC and/or GD. The yield of GLC was determined by ¹H NMR.**

| Catalyst | Structure | pK_{BH}+(MeCN) | GLC (%) | GLY (%) |
|---|---|---|---|---|
| Py | 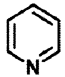 | 12.5 | 26 | 5 |
| PS | 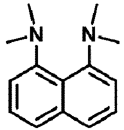 | 18.6 | 9 | 3 |
| TEA | 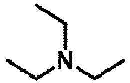 | 18.8 | 14 | 2 |
| TMG | 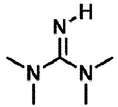 | 23.3 | 52 | 4 |
| DBU | 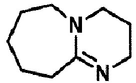 | 24.3 | 86 | 14 |
| TBD | 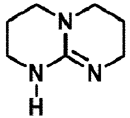 | 26.0 | 86 | 14 |
| BB | 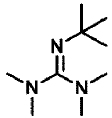 | 26.0 | 91 | 9 |
| P₁-*t*-Oct | 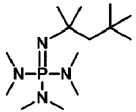 | 28.3 | 79 | 21 |
| VB | 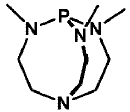 | 32.9 | 81 | 15 |

A control experiment without organocatalyst was performed to check that no conversion from GD to GLC occurred when no organocatalyst is present. Indeed, no conversion was detected, thereby excluding any potential catalytic activity arising from the reaction medium, e.g., the liquid solvent, which is in these experiments MEK.

For organocatalyst having a low pK_{aH}, that is, py, TEA and PS, the coupling of GD with CO₂ resulted in a low yield of GLC, namely of from 9 to 26%. Stronger bases, e.g., DBU and TBD, yielded GLC in much higher yields, that is, up to 86%, despite an increase of the hydrolysis product GLY, that had a yield of up to 14%. Barton's Base, i.e., BB, afforded the best compromise, with a high yield for GLC of 91% and with only a minor amount of GLY formed. Stronger bases than BB, that is, P₁-*t*-Oct and VB, significantly increased the formation of GLY.

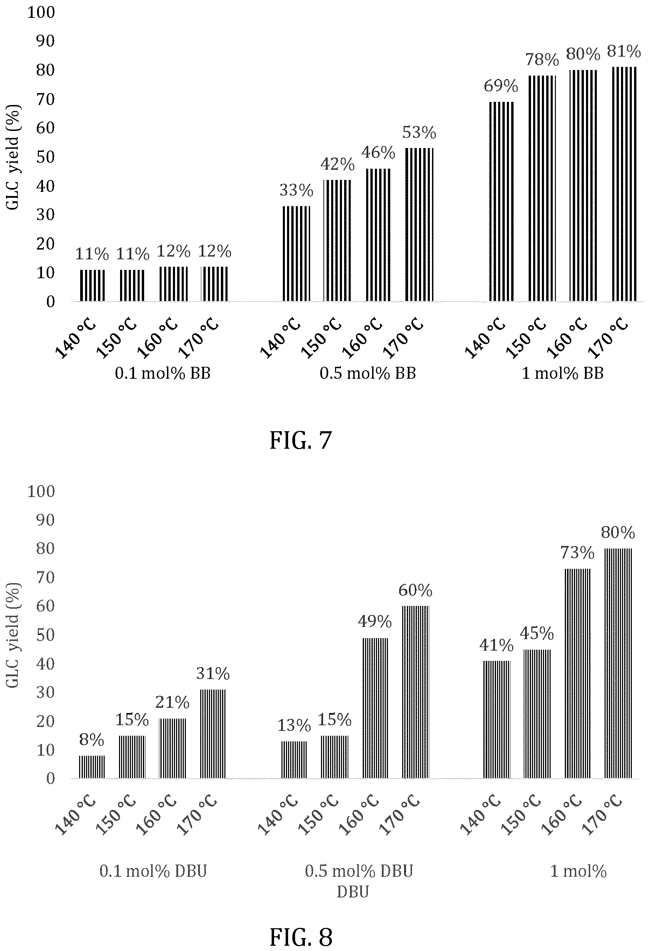

Subsequently, selecting BB as the most promising catalyst, further optimization was performed. Herein, different concentrations of BB, namely 0.1 mol%, 0.5 mol%, and 1 mol%, and different temperatures of the first channel, namely from 140°C to 170°C, were used. Reference is made to FIG. 7, wherein the GLC yield for the different temperatures and different concentrations of BB are summarized. It may be observed that all conditions led at least to some conversion of GD into GLC, although the best yields are achieved for the highest catalytic amount, i.e. 1 mol% (82%). It also became clear that for a given catalytic loading, the temperature only had a moderate impact on the formation of GLC. For instance, at 1 mol%, the yield toward GLC only increased from 69 to 82% when increasing the temperature of from 140 to 170 °C. When using a lower catalyst loading, the extent of the competitive formation of GLY became marginal, with yields in the range of from 6 to 9%, from 5 to 8%, and from 1 to 2%, at 1, 0.5 and 0.1 mol%, respectively. As such, a good yield for GLC was eventually found to be at 82% using only 1 mol% of the catalyst. Most notably, only a stoichiometric amount of CO₂ (1 equiv.) was used. Decreasing the catalytic loading to 0.5 and 0.1 mol% drastically decreased the conversion to 53 and 12%, respectively.

A similar screening of the impact of the catalytic loading of another organometallic catalyst, namely DBU, was also performed, given its high catalytic efficiency, in combination with a lower cost compared to Barton's Base. Reference is made to FIG. 8, wherein the GLC yield for the different temperatures and different concentrations of DBU are summarized. Conversion of GD to GLC was detected even at the lowest used catalytic loading. However, a more pronounced effect of the temperature on the reaction outcome was noticed with DBU. More specifically, increasing the temperature from 150 to 160 °C led to a significant increase in GLC yield, rising from 45 to 73% at 1 mol% of DBU. However, higher process temperatures with DBU were also accompanied with a higher formation of GLY (up to 10%).

### Scalability

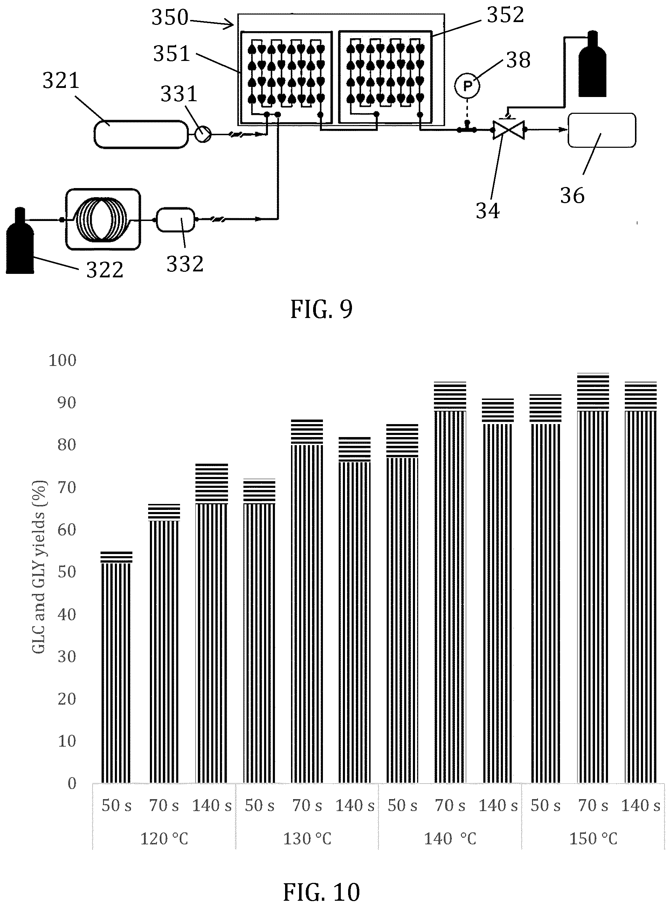

Progressive scalability of the process was evaluated using commercial mesofluidic glass reactors (Corning^{®} Advanced-Flow Reactors^{™}, AFR^{™}). Reference is made to FIG. 9 that is a schematic representation of a flow reactor comprising the lab scale mesofluidic system. First, an intermediate scalability trial was carried out using a first channel 350 that is a commercial lab scale reactor, i.e., a lab scale mesofluidic system (Corning^{®} AFR^{™} G1/LF squid equipped with 5 glass fluidic modules connected in series, 2.7 mL internal volume each, 13.5 mL of total internal volume). Herein, a first module 351 was used for mixing, so that the compounds were well-mixed in the other four modules 352 (of which only a single module is shown in FIG. 9) fluidically connected, downstream, to the first module 351. The first channel 350 was thermoregulated with a LAUDA^{®} XT Integral 280. The liquid feed was pumped from a liquid source 321 by a pump 331, that was a Syrris Asia Syringe Pump, into the first channel 350. The gaseous feed, i.e., carbon dioxide, was pumped, from a carbon dioxide cylinder 322, using a flow controller 332, i.e., a Bronkhorst EL FLOW Prestige mass flow controller, into the first channel 350. Downstream pressure was regulated with a dome-type backpressure regulator 34 (BPR-1000 Zaiput Flow Technologies). A homemade pressure sensor 38 (0-35 bar) was added upstream of the backpressure regulator 34 to monitor the stability of the pressure in the first channel 350. Reaction products were collected at an outlet 36.

Table 4 summarizes the results of the different experiments that were performed. Simultaneous reference is made to FIG. 10. Process temperatures of from 120 to 150 °C were assessed with a feed solution of GD (1.8 M in MEK) and 1 equiv. of CO₂ in the presence of 1 mol% of BB under 10 bar of counterpressure. Comparable trends to the microfluidic experiments were observed in the lab scale mesofluidic setup (Corning^{®} AFR^{™} G1/LF squid): increasing the temperature had a positive impact on the reaction's outcome. Three difference residence times were used (50, 70 and 140 s). For the same temperature, increasing the residence time from 50 to 140 s resulted in a greater conversion from GD to GLC. Similarly, for the same residence time, an increase of the temperature from 120 to 140 °C resulted in an increase in GLC yield. However, a further increase in temperature to 150 °C did not further improve conversion nor the GLC yield. These results may be explained by the lower concentration of dissolved CO₂ caused by the lower solubility of CO₂ at 150°C compared to that at 140°C. Indeed, the solubility of CO₂ is typically inversely correlated to the increase in the temperature of the system. In addition, at 150°C and 50 s, clogging of the glass first channel of the reactor was observed due to the polymerization of GD into oligo/polyethers. A maximum yield of GLC of 88% was obtained at a temperature of 140°C, a residence time of 70 s and a back pressure of 10 bar.

A further increase of both the temperature and residence time did not improve the selectivity toward GLC. At all flow rates, using a temperature in the glass first channel 350 of 150 °C led to the clogging of the reactor, i.e., of the first channel 350, most likely caused by the ring opening polymerization of GD. Typically, higher temperatures and higher flow rates promote clogging by polymerization. Additional experiments were also performed to study the influence of residence time independently of the mixing efficiency (which may be influenced by the liquid and gaseous flow rate), by removing fluidic modules from the first channel 350, while keeping all other process parameters identical, which are entries 8-12, performed at a liquid flow rate of 1.82 mL min⁻¹ and with a gaseous flow rate of 72.9 mL_{N} min⁻¹. Entries 8 to 12 clearly show that a decrease of the residence time has a moderate impact on the GLC yield. Indeed, when reducing the residence time from 70 (5 fluidic modules in series) to 41 s (3 fluidic modules in series), a loss of 10% in GLC yield was observed, whereas further shortening the residence time to 27 s (2 fluidic modules in series) and 13.5 s (1 fluidic module) lead to lower carbonate yields ranging at 63 and 31%, respectively.

**Table 4: Scalability trials for the carbonation of GD in a mesofluidic lab scale glass reactor of 13.5 mL for various temperature and residence times using 1 mol% of Barton's Base. All entries are performed at a liquid flow rate of 1.82 mL min⁻¹ and with a gaseous flow rate of 72.9 mL_{N} min⁻¹**

| Entry | T (°C) | Residence time (s) | GD conv. (%) | GLC yield (%) | GLY yield (%) |
|---|---|---|---|---|---|
| 1 | 120 | 50 | 55 | 52 | 3 |
| 2 | 120 | 70 | 66 | 62 | 4 |
| 3 | 120 | 140 | 76 | 66 | 10 |
| 4 | 130 | 50 | 71 | 66 | 6 |
| 5 | 130 | 70 | 86 | 80 | 6 |
| 6 | 130 | 140 | 82 | 76 | 6 |
| 7 | 140 | 50 | 84 | 77 | 6 |
| 8 | 140 | 70 | 95 | 88 | 7 |
| 9 | 140 | 54 | 91 | 82 | 8 |
| 10 | 140 | 41 | 87 | 78 | 9 |
| 11 | 140 | 27 | 69 | 63 | 6 |
| 12 | 140 | 13.5 | 34 | 31 | 3 |
| 13 | 140 | 140 | 93 | 85 | 8 |
| 14 | 150 | 50 | 92 | 85 | 7 |
| 15 | 150 | 70 | 97 | 88 | 9 |
| 16 | 150 | 140 | 95 | 88 | 7 |

Additional data were collected using DBU and TBD (1 mol%) as organocatalyst, under otherwise the same reaction conditions. In both cases, clogging of the first channel 350 was observed. DBU is known to easily form an adduct with CO₂, which displays a very poor solubility in most organic solvents, including MEK.

To check whether the adduct may be indeed formed, a synthesis procedure for forming an adduct of DBU with of CO₂ was performed. In a sealed 10 mL vial with a magnetic stirrer, 5 mL of neat DBU was reacted with an atmosphere of gaseous CO₂ at room temperature. The reaction is finished when a white precipitate is formed. The purification consists in the filtration under vacuum of the reactional medium and its washing with ethyl acetate. ¹H-NMR was used to confirm the successful synthesis of the adduct. The synthesis of the target compound was further confirmed in Lv, Min, et al. "Conversion of carbon dioxide into oxazolidinones mediated by quaternary ammonium salts and DBU." ChemCatChem 9 (2017) pages 4451-4455.

Using TBD as organocatalyst was unsuccessful as well, since it triggered the polymerization of GD, similarly to what was observed for BB.

The conditions were next transposed in a pilot scale mesofluidic reactor (Corning^{®} AFR^{™} G1 Reactor equipped with 7 glass fluidic modules connected in series, 8 mL internal volume each, 56 mL of internal volume). It was assumed that superior mixing efficiency and heat transfer within these commercial mesofluidic reactors compared to the PFA microfluidic prototype would further improve process conditions.

After successful experiments using the lab scale mesofluidic reactor, the carbonation process was attempted in a pilot mesofluidic reactor (Corning^{®} AFR^{™} G1) with BB (1 mol%). A similar flow reactor was used as for the lab scale mesofluidic reactor (cfr. FIG. 9), but for the first channel 350, seven fluidic modules of Corning^{®} AFR^{™} G1 having an internal volume of 8 mL per fluidic module, resulting in a total internal volume of 56, were used. As a pump 331, a Teledyne ISCO 500D dual pump system was used. Optimized conditions were found to be as follows: 1.8 M of GD in MEK, with 1 mol% BB, introduced from the liquid source 321 at a liquid flow rate of 16.18 mL min⁻¹, and CO₂ (gas, 1 equiv.) introduced from the CO₂ cylinder 322 at 648 mL_{N} min⁻¹, using a temperature of 140 °C, a pressure of 10 bar, and an estimated residence time of 28 s. The lab scale mesofluidic conditions were successfully transposed and further intensified with conversions of up to 85% within remarkable short residence times (60s: 85%, 43 s: 80%, 28 s: 78%, 21 s: 67%) at 140 °C. These performances simply outperform all conditions reported in the primary literature so far. The process can be complemented with Process Analytic Technology (APT), such as in-line IR between the first channel 350 and the outlet 36 for constant monitoring of the reaction products.

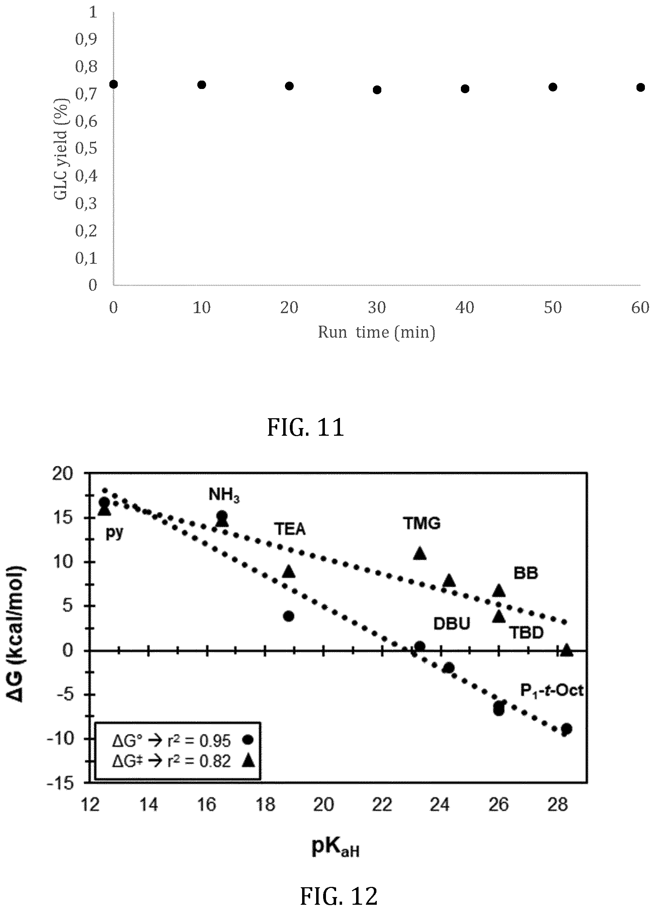

Reference is made to FIG. 11. An investigation of the stability of the carbonation of GD, i.e., of the yield of GLC, over a run of 1 hour using the Corning reactor G1 as the first channel was performed. Three aliquots of 1 min were collected every 10 minutes from the outlet and analysed using ¹H NMR Spectroscopy. Results showed that the production of GLC was constant over the entire duration of the experiment, with a yield that is constantly between 71 and 74% (standard deviation of 2%), indicating good stability of the process.

### Environmental considerations

Now, advantages with respect to sustainability of the process in accordance with embodiments of the present invention are summarized. The global efficiency was determined through the calculation of representative metrics. The Environmental factor (E-factor) was calculated with the inclusion of the catalyst, the solvent and the hydrolysis side product GLY in order to obtain the most accurate estimation of waste generation. While the production of fine chemicals is usually associated with E-factor values ranging between 5 and 50, this process is associated with an E-factor of 4.7, therefore highlighting its small environmental footprint. Moreover, according to the CHEM21 solvent selection guide, MEK is recommended as a green solvent and its favorable physico-chemical properties (boiling point: 80 °C) ease potential downstream recyclability. Lastly, this process meets several core principles of Green Chemistry: (a) renewable feedstocks may be used in stochiometric amount, (b) catalysis, (c) safe implementation and (d) energy efficiency, wherein the latter two are inherently related to continuous flow technology. The attractiveness of this process is emphasized by its high throughput and low footprint, which translates with a Space Time Yield (STY) of 2.7 kg h⁻¹ L⁻¹.

### Computations for the organocatalyzed coupling of GD and CO₂

The experimental results obtained above for processes in accordance with embodiments of the present invention were rationalized by DFT computations, without embodiments of the present invention being bound by the theory developed in the following section. For this, first, different pathways from the prior art are summarized, so that the reaction mechanism of the reaction in accordance with embodiments of the present invention may be compared with those of the prior art.

Conversion of epoxides toward the formation of cyclic carbonates can be achieved according to two different pathways.

A first, conventional synthetic pathway relies on the use of a binary catalytic system composed of a Lewis acid (or hydrogen bond donor) compound and an external nucleophile, according to the following mechanism: 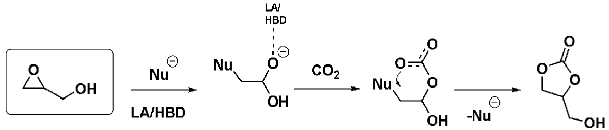 This mechanism contains (a) the activation of the epoxide by coordination or hydrogen bonding, (b) epoxide's ring opening via the external nucleophile to yield the stabilized (LA or H bond) alkoxide, (c) the subsequent fixation of CO₂ and (d) the intramolecular cyclization toward the cyclic carbonate.

A second, alternative pathway is as follows: 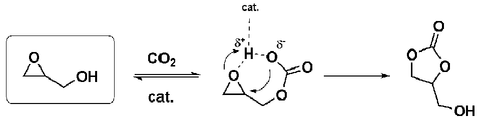 This pathway is driven by substrate activation in two main steps, namely (a) a preliminary intermolecular carbonation of the hydroxyl group of GD to yield an hemiester of carbonic acid via proton shuttling and (b) subsequent intramolecular nucleophilic attack to form GLC.

Rintjema, Jeroen, et al. Substrate-Controlled Product Divergence: Conversion of CO2 into Heterocyclic Products. Angewandte Chemie 128 (2016) pages 4040-4044 reported the carbonation of GD using a metal based complex of aluminium(III)-centered aminotriphenolate as Lewis acid. That report demonstrated that the implementation of a high temperature (50 °C) without a nucleophilic additive, enabled to solely trigger the alternative mechanism. In contrast, the use of an external nucleophile concomitantly triggered both paths. In order to rationalize this alternative mechanism, the same group performed a computational analysis combined to a kinetic study and the identification of catalytic intermediates via infrared spectroscopy. Considering that a first order dependence was determined for the Al catalyst and both reagents, computations were performed accordingly. The first step identified corresponds to the favourable coordination of OH in position β by the Al complex to generate a stable intermediate prior its deprotonation to yield the alkoxide intermediate. In absence of additive, the CO₂ fixation by the alkoxide and the epoxide opening are a stepwise process followed by the rate determining step, i.e., intramolecular cyclization toward GLC, displaying an overall barrier of activation at 46.2 kcal mol⁻¹. Interestingly, the use of a catalytic amount of water enabled to decrease ΔG^{‡} to 24.6 kcal mol⁻¹ due to (a) the stabilization of the alkoxide prior CO₂ addition to obtain an anion-CO₂-H₂O ensemble (intermediate) with a low activation barrier, (b) the facilitation of the formation of the hemiester species due to the stabilized ensemble and (c) the activation of the epoxide through hydrogen bonding. The reaction pathway was determined to be as follows:

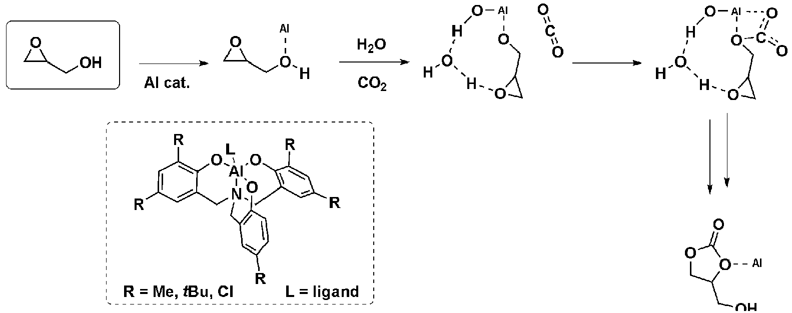

Therefore, the authors concluded that the CO₂ fixation and subsequent intramolecular cyclization resulted in a concerted mechanism, in comparison to the stepwise process in absence of water. Moreover, these calculations were supported by experiments in anhydrous and normal (wet) conditions, yielding 32% and 62% of GLC (50 °C, 4 h, 10 bar CO₂) respectively.

Similarly, Poolwong, Jitpisut, et al. "Simple Halogen-Free, Biobased Organic Salts Convert Glycidol to Glycerol Carbonate under Atmospheric CO2 Pressure." ChemSusChem 15 (2022) e202200765 describes the carbonation of neat GD in presence of sodium citrate as biobased catalyst under mild conditions (60 °C, 1 atm CO₂, 24 h), according to the following pathway: 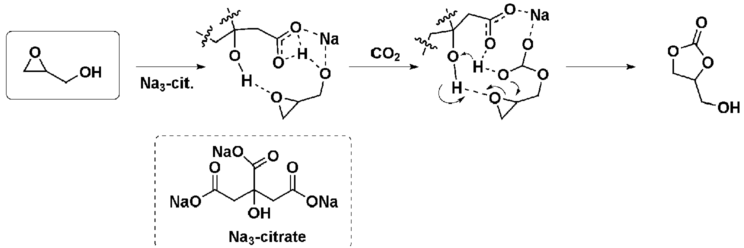 They emphasized the key role played by the basicity and the hydrogen bond acceptor ability of this organic salt. Mechanistic considerations enabled to confirm the triggering of the substrate activated path by implementing the same experimental conditions on ECH and styrene oxide (no conversion of substrate), therefore supporting the proton shuttling mechanism mediated by the free OH function. This substrate mediated pathway also enabled to convert more challenging substrates such as bicyclic epoxides while ensuring the control of their stereoselectivity. According to the catalyst conditions implemented and catalytic loading, the stereochemistry of the reactional output could be tuned to the selective synthesis of the desired diastereoisomeric compound.

Furthermore, an innovate organocatalyzed approach was reported toward the formation of tri-substituted carbonates, in Sopeña, Sergio, et al. "Organocatalyzed domino [3+2] cycloaddition/payne-type rearrangement using carbon dioxide and epoxy alcohols." Angewandte Chemie International Edition 57 (2018) pages 11203-11207. It involves a domino process composed of a substrate mediated carbonation with a subsequent dynamic equilibrium-controlled reaction, assimilable to a Payne-type rearrangement toward the targeted highly substituted carbonate. Additional works dealt with new catalytic roads and synthetic pathways still relying on the unique reactivity of epoxides bearing β positioned OH group toward the synthesis of six-membered cyclic carbonates. (Qiao, Chang, et al. "A Novel Catalytic Route to Polymerizable Bicyclic Cyclic Carbonate Monomers from Carbon Dioxide." Angewandte Chemie 134 (2022) e202205053, and Qiao, Chang, et al. "Organocatalytic trapping of elusive carbon dioxide-based heterocycles by a kinetically controlled cascade process." Angewandte Chemie 132.42 (2020): 18604-18609.)

In what follows, the experimental results for processes in accordance with embodiments of the present invention are rationalized by DFT computations. Herein, the coupling reaction was computed for a selection of representative homogeneous organic bases that may be used in processes in accordance with embodiments of the present invention (py, TEA, TMG, DBU, TBD, BB, P₁-*t*-Oct). NHs was added to the scope as a simple, reference nitrogen-containing base.

DFT computations was performed using the Gaussian 16 (Revision C.01) software package. Geometry optimizations were carried out at the B3LYP-D3/6-31+G^{∗} level of theory. The SMD (Solvent Model Based on Density) method was used to model solvent effects using the built-in parameters in Gaussian 16, parameters for 2-butanone (MEK; dielectric constant ε = 18.246). All possible stationary points were localized at the B3LYP-GD3BJ/6-31+G^{∗} level of theory. Stationary points were characterized by frequencies computations (local minima with only real frequencies and transition states with one imaginary frequency), and a systematic attempt to locate the lowest energy form was made. Transition states were verified by following the intrinsic reaction coordinates (IRC). Electronic energies were computed at the M08HX/6-311++G^{∗∗} level whereas solvation and thermal energies were computed at the B3LYP-GD3BJ/6-31+G^{∗} level and corrected for concentration, temperature and quasi-harmonic factors at 413.15 K using the GoodVibes Python script.

The simplified mechanism for the coupling, i.e., reaction, of GD with CO₂ in the presence of a selection of nitrogen-containing catalysts is as follows:

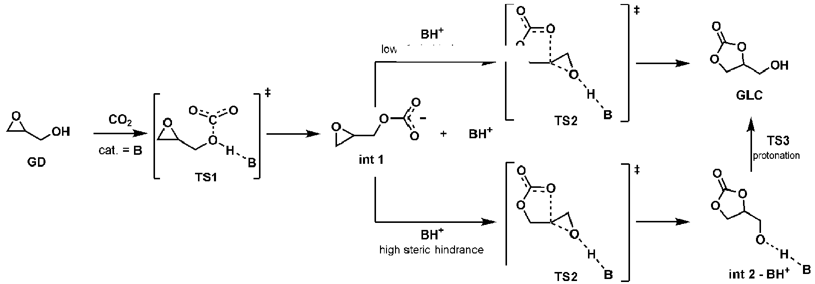

The first step is an intermolecular concerted reaction involving the organocatalyst, the hydroxyl group of GD, and CO₂, which leads to an intermediate "int 1". This step proceeds through a low-lying transition state (TS1) with activation barriers ranging from 16 to 0.1 kcal mol⁻¹ depending on the catalyst, indicating that the reaction of GD with CO₂ is fast. The activation barriers for P₁-*t*-Oct, TBD, BB, DBU, TEA, TMG, NH₃, and py are respectively 0.1, 3.9, 6.8, 7.9, 9.0, 11.0, 14.7, and 16.0 kcal mol⁻¹. This wide range of activation barriers as a function of the catalyst can be correlated with the extended range of their respective p*K*_{aH}.

Reference is made to FIG. 12, which shows a dependence of the calculated Free Gibbs energy of reaction Δ*G*° and the calculated activation barrier Δ*G*^{‡} as a function of p*K*_{aH} (in acetonitrile; values from literature) of a range of organocatalysts for the first intermolecular step. It appears that the activation barrier (Δ*G*^{‡}) decreases with increasing basicity of the catalyst.

From these correlations, it became clear that a strongly basic catalyst may promote a fast reaction between CO₂ and GD. Moreover, in order to determine a potential influence of water as cocatalyst, experiments in anhydrous (dry solvent) and normal conditions were performed. Comparison of GLC yields highlighted unsignificant differences (2 - 4%), showing that water barely influences the reaction, therefore supporting the mechanism shown above. The explanation lies with the fact that the addition of water plays the same role as that of the conjugated acid, that is, it stabilizes the alkoxide intermediate and activates the epoxide in order to facilitate int 1 nucleophilic attack, thus rendering its addition unnecessary.

Regarding the thermodynamics of the process, the high stability of CO₂ may be overcome due to the simultaneous favorable acid-base reaction between the catalyst and the alcohol function of GD. This assumption is supported by a calculated negative value of Calculated Free Gibbs energy of reaction Δ*G*° for several of the catalysts, which is quite surprising considering the expected low reactivity of CO₂. Indeed, each of the catalysts that had the most promising results in the experiments above, that is, DBU, TBD, BB and P₁-*t*-Oct, are found to have a negative Δ*G*°, thus facilitating a fast reaction between GD and CO₂. For the weaker bases, Δ*G*° > 0. Int 1 obtained from P₁-t-Oct, TBD, BB, DBU, TEA, TMG, NH₃, and py, have a Δ*G*° of respectively -8.9, -6.8, -6.3, -1.9, 0.4, 3.9, 15.2, and 16.2 kcal mol⁻¹, respectively.

To confirm the critical role of the OH group of GD, experiments for the carbonation of ECH, and the carbonation of t-butyl glycidyl ether, were performed using similar experimental conditions as were used for the carbonation of GD, using BB as organocatalyst. For neither ECH nor t-butyl glycidyl ether, a detectable conversion toward GLC was achieved. An additional experiment, under similar conditions, aiming at the carbonation of a mixture of GD/ECH (ratio 2.25:1), confirmed the selectivity and mechanistic features of this reaction: GD was converted to GLC, whereas ECH did not react.

The second step, i.e., the intramolecular cyclization step from "int 1" toward GLC, is promoted by the activation of the oxirane moiety of "int 1" by the conjugated acid of the catalyst (produced in step 1) through a proton shuffle. They are, however, two distinct mechanisms depending on the type of catalyst, and more specifically the steric hindrance provided by the catalyst. Catalysts with a lower steric hindrance (such as py, TEA, TMG and DBU) may directly lead to the final products (GLC and the regenerated active catalytic species) without the formation of any intermediate. It involves a concerted TS structure where both the intramolecular cyclization (TS2, event 1) and the proton transfer to the alkoxide (TS2, event 2) occur. IRC computations indeed indicated an atypical high asynchronicity between these two events.

In contrast, sterically highly hindered bases (such as BB and P₁-*t*-Oct) proceed through an alternative stepwise pathway with the formation of an intermediate species ("int 2"), followed with its protonation in a second step. The intramolecular cyclization occurs first, where the oxirane ring is activated through a H-bonding type of interaction with the conjugated acid of the catalyst, yielding "int 2". The latter intermediate species is then protonated to give GLC with the concomitant recovery of the catalyst. The Δ*G*^{‡} associated with the intramolecular cyclization is 27.4 kcal mol⁻¹ without catalyst and range from 24.3 to 18.8 kcal mol⁻¹, depending on the catalyst. It is respectively 18.8, 19.9, 21.5, 22.7, 23.2, 23.6, 24.3, and 24.9 for NH₃, py, TEA, P₁-t-Oct, DBU, BB, TBD, and TMG.

In the case of TBD, the stepwise pathway seems to be favored due to the symmetry of its protonated form. In any case, the evolution from a concerted to a stepwise mechanism for the intramolecular cyclization/protonation events expresses an extreme case of asynchronicity associated with the catalyst's structural features (either steric or symmetric). The uncatalyzed intramolecular cyclization was also investigated and was deemed irrelevant due to its higher Δ*G*^{‡} (27.4 kcal mol⁻¹) compared to catalyzed path. Moreover, the presence of the catalyst shifts the equilibrium toward the formation of the products by drastically lowering the variation of Gibbs free energy.

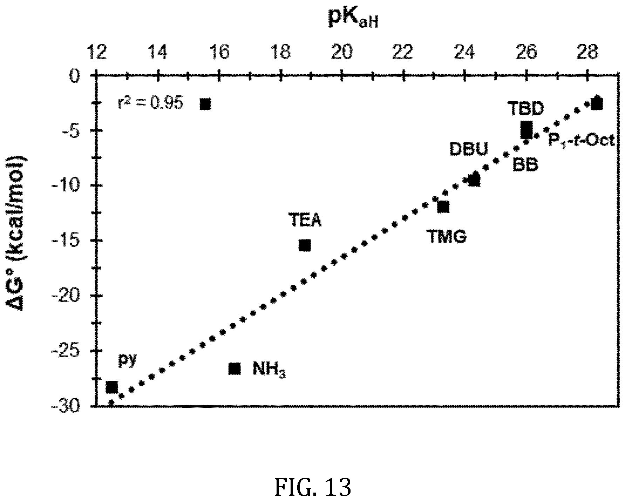

Reference is made to FIG. 13, which shows a dependence of the calculated Free Gibbs energy of reaction Δ*G*° and the calculated activation barrier Δ*G*^{‡} as a function of p*K*_{aH} (in acetonitrile; values from literature) of the organocatalyst for the second intermolecular step. The thermodynamics of the second step are again dictated by the acid-base equilibrium, as shown by the linear relationship in FIG. 13.

In conclusion, the role of the catalyst's basicity is of importance in the first reaction step. Indeed, a high p*K*_{aH} ensures a rapid and favorable CO₂ fixation thanks to the formation of a highly stable conjugated acid along with a linear carbonate intermediate. The rate of the second step is drastically enhanced by the presence of the protonated base, although it remains rate-determining.

Given the fast kinetics of both steps, the overall rate of the carbonation is likely limited by the solubility of CO₂ in the reaction medium, therefore justifying both the selection of MEK as reaction medium and a high mass transfer technological solution.

### Summary of chemicals

Chemicals that were used in the above Examples are summarized in Table 5.

**Table 5: The reagents and solvents are purchased from commercial sources; these compounds are used without purification.**

| Solvents | Purity (%) | CAS number | Supplier |
|---|---|---|---|
| Ethanol (absolute) | >99% | 64-17-5 | VWR |
| Ethyl acetate | >99.5% | 141-78-6 | VWR |
| Methyl ethyl ketone | >99% | 78-93-3 | VWR |
| D3-acetonitrile | >99.8% | 2206-26-0 | Eurisotop |
| Petroleum spirit 40-60 °C | / | 8032-32-4 | VWR |
| Chemicals | Purity (%) | CAS number | Supplier |
| 1,2,3-Propanetriol (glycerol) | 99% | 56-81-5 | ABCR |
| Hydrochloric acid 36 wt.-% | / | 7647-01-0 | Merck |
| 2-Chloropropane-1,3-diol | ≥97% | 497-04-1 | Carbosynth Ltd. |
| 3-Chloropropane-1,2-diol | 99% | 96-24-2 | Acros Organics |
| 1,3-Dichloropropan-2-ol | 99% | 96-23-1 | Acros Organics |
| 2,3-Dichlorpropan-1-ol | ≥98% | 616-23-9 | TCI |
| 2,3-Epoxy-1-propanol | 96% | 556-52-5 | Acros Organics |
| 2-(Chloromethyl)oxirane | 99% | 106-89-8 | Acros Organics |
| Acetic Acid | 99% | 64-19-7 | VWR |
| Sodium Hydroxide | 98.5% | 1310-73-2 | Acros Organics |
| 4-(Hydroxymethyl)-1,3-dioxolan-2-one | >90% | 931-40-8 | Sigma-Aldrich |
| 1,8-Diazabicyclo[5.4.0]undec-7-ene (DBU) | >99% | 6674-22-2 | Merck/Sigma Aldrich |
| 2,8,9-Trimethyl-2,5,8,9-tetraaza-1-phosphabicyclo[3.3.3]undecane (Verkade's base) | / | 120666-13-9 | Merck/Sigma Aldrich |
| 1,1,3,3-Tetramethylguanidine (TMG) | 99% | 80-70-6 | Merck/Sigma Aldrich |
| 2-tert-Butyl-1,1,3,3-tetramethylguanidine (Barton's base) | ≥97% | 29166-72-1 | Merck/Sigma Aldrich |
| tert-Octyliminotris(dimethylamino)phosphorane (P1-t-Oct) | ≥97% | 161118-69-0 | Merck/Sigma Aldrich |
| 1,5,7-Triazabicyclo[4.4.0]dec-5-ene (TBD) | 98% | 5807-14-7 | Merck/Sigma Aldrich |
| Triethylamine | 99% | 121-44-8 | VWR |
| Pyridine | 99% | 110-86-1 | Across |

### Summary of components of the microfluidic setups

All microfluidic setups were assembled with commercially available components. Details of the components are summarized in Table 6.

Pumps and mass flow controllers: Chemyx Fusion 6000^{®} High Force syringe pumps equipped with stainless steel syringes (6 or 20 mL) with Dupont^{™} Kalrez^{®} Spectrum^{™} AS-568 O-rings (0.549 x 0.103") or Asia syringe pumps (Syrris) using Asia Blue Syringes (1 mL / 2.5 mL) were used to carry out the liquid feeds. Gaseous CO₂ was handled with a Bronkhorst EL FLOW Prestige mass flow controller.

Microfluidic prototype (PFA coil reactors): Coil reactors, collections and feed lines were constructed with high purity PFA tubing (1.58 mm outer diameter and 800 µm of internal diameter).

Connectors, ferrules and mixers: Unions, arrowhead and static mixers, PEEK connectors and ferrules were purchased from IDEX/Upchurch; details can be found in Table 6.

Check valves: Check valves (with PEEK check valve holders) were inserted on feed lines downstream the pumps/MFC and were purchased from IDEX/Upchurch Scientific.

Back pressure regulators: A dome-type BPR (BPR-10 from Zaiput Flow Technologies) was inserted downstream the microfluidic prototype. Cracking pressure setpoints were regulated with compressed nitrogen gas (AirLiquide).

Thermoregulatory devices: Thermoregulation of PFA coil reactors was performed with a Heidolph^{™} MR Hei-Tec^{®} equipped with a Pt-1000 temperature sensor.

**Table 6: Connectors, ferules and unions**

| Part | Details | Vendor | Reference |
|---|---|---|---|
| Connectors | One-Piece Fingertight, PEEK, 10-32 Coned, for 1/16" OD | IDEX/Upchurch Scientific | F-120X |
| | Super Flangeless Nuts, natural PEEK 1/4-28 thread for 1/16" OD tubing | IDEX/Upchurch Scientific | P-255X |
| | Super Flangeless Ferrule Tefzel (ETFE) and SS ring 1/4-28 thread for 1/16" OD tubing | IDEX/Upchurch Scientific | P-259X |
| Mixers | T-mixer, natural PEEK 1/4-28 thread for 1/16" o.d. tubing, 0.02" through hole | IDEX/Upchurch Scientific | P-712 |
| | High Pressure Static Mixing Tee (arrowhead), PEEK, 10-32 Coned thread for 1/16" o.d. tubing, 0.02" through hole, embedded with a UHMWPE Frit | IDEX/Upchurch Scientific | U-466 |
| Tubing | High-purity PFA tubing, 1.58 mm outer diameter, 750 µm internal diameter | VICI (Valco Ins. Co. Inc.) | JR-T-4002-M25 |
| | High-purity 1/8" and 1/4" PFA tubing, including appropriate PFA connections | Swagelok | PFA-T2-030-100 |
| | | | PFA-T4-047-100 |
| Check-valve | Check-valve inline cartridge 1.5 psi | IDEX/ Upchurch Scientific | CV-3001 |
| Dome-type BPR | Dome-type BPR, metal-free, with adjustable set point | Zaiput Flow Techn. | BPR-10 |

### Summary of components of the mesofluidic setups

Pumps: A Teledyne ISCO 500D dual syringe pump system was used to continuously handle the liquid feeds.

Mass flow controller: Gaseous CO₂ was handled with a Bronkhorst EL FLOW Prestige mass flow controller.

Mesofluidic reactors: Scalability trials relied on commercial mesofluidic glass reactors (Corning^{®} Advanced-Flow Reactors^{™}). Intermediate scalability trials were carried out with a Corning^{®} AFR^{™} G1/LF squid equipped with 5 glass fluidic modules connected in series (2.7 mL internal volume each, 13.5 mL of total internal volume). Pilot scale trials were carried out with a Corning^{®} AFR^{™} G1 Reactor equipped with 7 glass fluidic modules connected in series (8 mL internal volume each, 56 mL of internal volume). All fluidic modules were equipped with standard Swagelok connectors and PFA tubing (1/4").

Back-pressure regulator: A dome-type BPR (Zaiput Flow Technologies - BPR-1000) was inserted downstream the mesofluidic setups to set the system pressure (connected to a compressed nitrogen tank).

Thermoregulatory device: The fluidic modules were thermoregulated at working temperature with a LAUDA Integral XT 280 thermostat (LAUDA Therm 180 silicone oil).

It is to be understood that although preferred embodiments, specific constructions and configurations, as well as materials, have been discussed herein for devices according to the present invention, various changes or modifications in form and detail may be made without departing from the scope of this invention. Steps may be added or deleted to methods described within the scope of the present invention.

## Claims

1. A continuous-flow process for the production of glycerol carbonate, wherein the method comprises reacting, in a liquid solvent in a first channel of a flow reactor, glycidol with carbon dioxide in the presence of a homogeneous nitrogen-containing organocatalyst having a p*K*ₐ of at least 15 when measured in acetonitrile,
wherein the reaction is carried out at a temperature of at least 90 °C, and
wherein the first channel has an inner hydraulic diameter of from 100 to 2000 µm, and
a pressure in the first channel is adapted to keep the solvent liquid.

2. The process according to claim 1, wherein the organocatalyst has the following chemical formula: 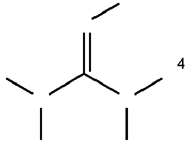 wherein
• R¹ is selected from: H, alkyl, heteroalkyl, aryl, heteroaryl, and benzyl,
• R² and R³ are either independently selected from H, alkyl, heteroalkyl, aryl, heteroaryl, and benzyl, or the radicals R² and R³, together with the nitrogen atom to which R² is bonded, the nitrogen atom to which R³ is bonded, and the carbon atom linking both said nitrogen atoms, form a five- to eight-membered ring, and
• R⁴ is independently selected from H, alkyl, heteroalkyl, aryl, heteroaryl, and benzyl, while R⁵ is independently selected from alkyl, heteroalkyl, aryl, heteroaryl, and benzyl, or the radicals R⁴ and R⁵, together with the nitrogen atom to which R⁴ is bonded, the nitrogen atom to which R⁵ is bonded, and the carbon atom linking both said nitrogen atoms, form a five- to eight-membered ring,
with the proviso that R² and R³ on one hand and R⁴ and R⁵ on the other are not simultaneously part of a ring.

3. The process according to claim 2, wherein
• R¹ is selected from H and methyl,
• R² and R³ are both methyl or the radicals R² and R³, together with the nitrogen atom to which R² is bonded, the nitrogen atom to which R³ is bonded, and the carbon atom linking both said nitrogen atoms, form a five-membered ring, and
• R⁴ is independently selected from H and methyl while R⁵ is independently selected from a C₁-C₄ alkyl and a phenyl, or the radicals R⁴ and R⁵, together with the nitrogen atom to which R⁴ is bonded, the nitrogen atom to which R⁵ is bonded, and the carbon atom linking both said nitrogen atoms, form a six-membered ring.

4. The process according to claim 2 or 3, wherein the organocatalyst is Barton's base.

5. The process according to any one of the preceding claims, wherein the catalyst has a p*K*ₐ of at least 20 when measured in acetonitrile.

6. The process according to claim 5, wherein the organocatalyst has a p*K*ₐ of from 20 to 30 when measured in acetonitrile.

7. The process according to any one of the preceding claims, wherein the catalyst is present in an amount of from 0.1 to 20 mol-%, with respect to the total amount, in mole, of the catalyst, the glycidol, and the carbon dioxide.

8. The process according to claim 7, wherein the catalyst is present in an amount of from 0.5 to 10 mol-%, preferably from 1 to 5 mol%, with respect to the total amount, in mole, of the catalyst, the glycidol, and the carbon dioxide.

9. The process according to any one of the preceding claims, wherein the solvent is selected from 2-butanone and n-butanol.

10. The process according to any one of the preceding claims, wherein the flow reactor has a mixing efficiency defined by an overall mass transfer coefficient k_{L}×a, of at least 100 × 10⁻⁴ s⁻¹, where k_{L} is the mass transfer coefficient in m/s and a is the effective interfacial area in m²/m³.

11. The process according to any one of the preceding claims, wherein the reaction is carried out at a temperature of from 100 °C to 149 °C, preferably from 110 °C to 148 °C, more preferably from 120°C to 145°C.

12. The process according to any one of the preceding claims, wherein a pressure in the first channel is at least 5 bar, preferably at least 10 bar, such as from 10 to 20 bar.

13. The process according to any one of the preceding claims, wherein the carbon dioxide is introduced in the first channel at a flow rate such that from 0.5 to 1.5, preferably from 0.9 to 1.5, more preferably from 1.0 to 1.5 equivalents of carbon dioxide is present with respect to glycidol.

14. The process according to any one of the preceding claims, further comprising a step, prior to the reaction of glycidol with carbon dioxide, of producing the glycidol by:
reacting glycerol with hydrochloric acid in a second channel of the flow reactor, wherein the second channel has an inner hydraulic diameter of from 100 to 2000 µm, thereby forming a reaction mixture, then
reacting the reaction mixture with sodium hydroxide in a third channel of the flow reactor, wherein the third channel has an inner hydraulic diameter of from 100 to 2000 µm, thereby producing the glycidol.

15. A flow reactor for performing the continuous-flow process according to any one of the preceding claims, the flow reactor comprising:
sources of compounds, comprising:
a source of the liquid solvent,
a source of the homogeneous nitrogen-containing organocatalyst,
a source of the carbon dioxide, and
a source of the glycidol,
a first channel, having an inner hydraulic diameter of from 100 to 2000 µm defining a fluidic pathway,
means for providing the compounds into the fluidic pathway, said means comprising a gas flow controller for allowing the carbon dioxide to enter directly in the fluidic pathway at a controlled flow rate,
means for setting the temperature in the first channel to a temperature of at least 90 °C, and
means for pressurizing the first channel sufficiently to keep the solvent in the first channel liquid.
